(19) Europäisches Patentamt
European Patent Office
Office européen des brevets

(11) EP 0 648 103 B1

(12) EUROPÄISCHE PATENTSCHRIFT

(45) Veröffentlichungstag und Bekanntmachung des Hinweises auf die Patenterteilung:
08.04.1998 Patentblatt 1998/15

(21) Anmeldenummer: 93914724.5

(22) Anmeldetag: 24.06.1993

(51) Int. Cl.$^6$: A61K 7/06

(86) Internationale Anmeldenummer:
PCT/EP93/01625

(87) Internationale Veröffentlichungsnummer:
WO 94/01076 (20.01.1994 Gazette 1994/03)

(54) HAARBEHANDLUNGSMITTEL

HAIR CONDITIONER

AGENT DE TRAITEMENT CAPILLAIRE

(84) Benannte Vertragsstaaten:
AT BE CH DE DK ES FR GB GR IE IT LI NL PT SE

(30) Priorität: 03.07.1992 DE 4221914
29.09.1992 DE 4232512
13.10.1992 DE 4234405
13.10.1992 DE 4234413

(43) Veröffentlichungstag der Anmeldung:
19.04.1995 Patentblatt 1995/16

(73) Patentinhaber:
Henkel Kommanditgesellschaft auf Aktien
40191 Düsseldorf (DE)

(72) Erfinder:
• SEIDEL, Kurt
D-4000 Düsseldorf 13 (DE)
• MÜLLER, Reinhard
D-5140 Erkelenz 7 (DE)
• HOLLENBERG, Detlef
D-4006 Erkrath (DE)
• PRIEBE, Christian
D-5603 Wülfrath (DE)

(56) Entgegenhaltungen:
WO-A-91/03229          WO-A-92/17153
WO-A-93/05756          GB-A- 2 063 671

**Beschreibung**

Die Erfindung betrifft Zubereitungen zur Haarbehandlung mit einer speziellen Kombination von Polymeren.

Die Reinigung und Pflege der Haare ist ein wichtiger Bestandteil der menschlichen Körperpflege. Sowohl die Reinigung der Haare mit Shampoos als auch die dekorative Gestaltung der Frisur beispielsweise durch Färben oder Dauerwellen sind Eingriffe, die die natürliche Struktur und die Eigenschaften der Haare beeinflussen. So können anschließend an eine solche Behandlung beispielsweise die Naß- und Trockenkämmbarkeit des Haares, der Halt und die Fülle des Haares unbefriedigend sein oder die Haare einen erhöhten Spliß aufweisen.

Es ist daher seit langem üblich, die Haare einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung Kämmbarkeit, Halt und Fülle der Haare verbessert und die Splißrate verringert.

Zusätze von kationischen Polymeren zu Haarbehandlungsmitteln führen in der Regel zu einer verbesserten Naß- und Trockenkämmbarkeit; Zusätze von amphoteren oder zwitterionischen Polymeren zu einer stark verbesserten Naßkämmbarkeit, während die Trockenkämmbarkeit meist nur wenig beeinflußt wird.

Weitere positive Effekte lassen sich häufig durch Kombination von mehreren Wirkstoffen aus unterschiedlichen Klassen erzielen. So ist beispielsweise aus der deutschen Offenlegungsschrift DE-A1-30 44 738 (GB-A-2063671) bekannt, Haarbehandlungsmittel mit einer Mischung aus amphoteren und kationischen Polymeren zu versehen. Gleichwohl die Druckschrift eine Vielzahl kommerzieller Polymere nennt, enthält sie keine Angaben über spezielle Vorteile spezifischer Polymerkombinationen.

Weiterhin sind aus der nicht vorveröffentlichten WO-A-9305756 Zubereitungen zur Haarbehandlung bekannt, die zwitterionische Polymerisate in Kombination mit bestimmten Pyrrolidoncarbonsäuren bzw. deren Salzen enthalten. Diese Zubereitungen können gewünschtenfalls weiterhin kationische Polymere, z. B. quaternisierte Celluloseether, enthalten.

Während die Verbesserung der Naßkämmbarkeit, d.h. eine Erniedrigung der Naßkämmarbeit in allen Fällen gewünscht wird, sind die Verhältnisse bei der Trockenkämmbarkeit komplizierter. Niedrige Kämmarbeits-Werte charakterisieren zwar eine Verbesserung der Kämmbarkeit; wird die Kämmarbeit aber zu sehr erniedrigt, so verliert das Haar Fülle und Halt, so daß sich im Extremfall bestimmte Frisuren nicht mehr aufbauen lassen. Daher kann, vor allem bei komplexeren Frisuren, eine in bestimmten Grenzen erhöhte Trockenkämmarbeit durchaus gewünscht sein, um den Halt der Frisur zu verbessern.

Es wurde nun überraschenderweise gefunden, daß Mittel mit einer Kombination aus zwitterionischen Polymeren und bestimmten kationischen Polymeren die Eigenschaften der Haare in überraschend guter Weise verbessern.

Gegenstand der Erfindung sind daher wäßrige Zubereitungen zur Behandlung von Haaren, enthaltend neben üblichen kosmetischen Bestandteilen eine Kombination von kationischen und zwitterionischen Polymeren, dadurch gekennzeichnet, daß die kationischen Polymere Cellulose-Derivate sind und keine Imidazoliniumgruppen enthalten und sich die zwitterionischen Polymeren im wesentlichen zusammensetzen aus

α) Monomeren mit quartären Ammoniumgruppen der allgemeinen Formel (I),

$$R^1\text{-}CH{=}CR^2\text{-}CO\text{-}Z\text{-}(C_nH_{2n})\text{-}N^{(+)}R^3R^4R^5\ A^{(-)} \tag{I}$$

in der $R^1$ und $R^2$ unabhängig voneinander stehen für Wasserstoff oder eine Methylgruppe und $R^3$, $R^4$ und $R^5$ unabhängig voneinander für Alkylgruppen mit 1 bis 4 Kohlenstoff-Atomen, Z eine NH-Gruppe oder ein Sauerstoffatom, n eine ganze Zahl von 2 bis 5 und $A^{(-)}$ das Anion einer organischen oder anorganischen Säure ist und

β) monomeren Carbonsäuren der allgemeinen Formel (II),

$$R^6\text{-}CH{=}CR^7\text{-}COOH \tag{II}$$

in denen $R^6$ und $R^7$ unabhängig voneinander Wasserstoff oder Methylgruppen sind, oder Alkali-, Erdalkali-, Aluminium- oder Ammoniumsalzen dieser Säuren.

Bei den erfindungsgemäß verwendbaren kationischen Polymeren handelt es sich um Cellulose-Derivate.

Eine Klasse dieser Cellulose-Derivate stellen die Substanzen dar, die durch Modifizierung eines Cellulose-Grundkörpers mit kationischen Gruppen erhalten werden.

Beispiele für Cellulose-Grundkörper sind Cellulose, Methylcellulose, Ethylcellulose, Hydroxyethylcellulose, Hydroxypropylcellulose und Methylhydroxypropylcellulose.

2

Übliche kationische Gruppen sind Gruppen mit quartären Stickstoff- oder Phosphoratomen. Gruppen mit quartären Stickstoffatomen sind dabei bevorzugt. Die quartären Stickstoffatome können dabei sowohl 4 unterschiedliche oder z.T. gleiche Substituenten tragen, als auch Teil eines Ringsystems sein. Bevorzugte Substituenten sind Alkylgruppen mit 1 bis 4 Kohlenstoffatomen, Arylgruppen, Alkylarylgruppen, Cycloalkylgruppen, Alkyoxyalkylgruppen und Alkoxyarylgruppen. Trialkylammoniumgruppen, insbesondere Trimethylammoniumgruppen, sind besonders bevorzugt. Die kationischen Gruppen können entweder direkt oder über Zwischengruppen mit dem Cellulose-Gerüst verbunden sein. Es sind solche Polymeren bevorzugt, bei denen die kationischen Gruppen über Alkylketten oder Alkoxyalkylgruppen mit dem Cellulosegrundgerüst verbunden sind. Bevorzugte Gegenionen zu den kationischen Gruppen sind Halogenid-, insbesondere Chlorid-, und Methosulfatgruppen.

Beispiele für solche Verbindungen sind die in der US-Patentschrift 3,472,840 beschriebenen Verbindungen, die unter der Bezeichnung Polymer JR$^R$ im Handel erhältlich sind.

Eine weitere Klasse von kationischen Cellulose-Derivaten bilden solche Verbindungen, die aus kationischen und nichtionischen Einheiten aufgebaut sind.

Die kationische Einheiten können entsprechend den oben genannten kationischen Cellulose-Derivaten aufgebaut sein.

Die kationischen Einheiten können weiterhin Verbindungen sein, die neben mindestens einer kationischen Gruppe mindestens eine polymerisierbare Gruppe enthalten. Die polymerisierbare Gruppe ist bevorzugt eine Vinylgruppe. Die kationischen Gruppen sind bevorzugt die oben genannten Ammoniumgruppen. Solche kationischen Einheiten sind beispielsweise Diallyldimethylammoniumchlorid, Methyacrylamidopropyltrimethylammoniumchlorid, mit Diethylsulfat quaterniertes Dimethylaminomethacrylat und mit Diethylsulfat quaterniertes Dimethylaminoethylmethacrylat.

Nichtionische Einheiten sind beispielsweise Cellulose, Methylcellulose, Ethylcellulose, Hydroxyethylcellulose, Hydroxypropylcellulose, Methylhydroxypropylcellulose, Vinylpyrrolidon, Vinylacetat, Acrylamid, Methacrylamid, Methylacrylat, Ethylacrylat, Methylmethacrylat und Ethylmethacrylat. Hydroxyethylcellulose ist eine bevorzugte nichtionische Einheit.

Beispiele für solche Copolymere sind die unter der Bezeichnung Celquat$^R$ erhältlichen Verbindungen.

Kationische Polymere, die Copolymere aus mindestens einer kationischen Einheit und mindestens einer nichtionischen Einheit sind, sind im Rahmen der erfindungsgemäßen Lehre bevorzugt. Besonders bevorzugt sind solche Copolymere, bei denen die nichtionische Einheit Hydroxyethylcellulose ist.

Celquat$^R$L 200, ein Copolymeres aus Hydroxyethylcellulose und Diallyldimethylammoniumchlorid, ist ein besonders bevorzugtes kationisches Polymer.

Unter "zwitterionischen Polymeren" werden solche Polymeren verstanden, die im Molekül quartäre Ammoniumgruppen und $-COO^-$ oder $-SO_3^-$-Gruppen enthalten.

Erfindungsgemäß werden zwitterionische Polymere eingesetzt, die sich im wesentlichen zusammensetzen aus

($\alpha$) Monomeren mit quartären Ammoniumgruppen der allgemeinen Formel (I),

$$R^1\text{-CH=CR}^2\text{-CO-X-}(C_nH_{2n})\text{-N}^{(+)}R^3R^4R^5\ A^{(-)} \tag{I}$$

in der $R^1$ und $R^2$ unabhängig voneinander stehen für Wasserstoff oder eine Methylgruppe und $R^3$, $R^4$ und $R^5$ unabhängig voneinander für Alkylgruppen mit 1 bis 4 Kohlenstoff-Atomen, X eine NH-Gruppe oder ein Sauerstoffatom, n eine ganze Zahl von 2 bis 5 und $A^{(-)}$ das Anion einer organischen oder anorganischen Säure ist
und

($\beta$) monomeren Carbonsäuren der allgemeinen Formel (II),

$$R^6\text{-CH=CR}^7\text{-COOH} \tag{II}$$

in denen $R^6$ und $R^7$ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen.

Ganz besonders bevorzugt sind solche Polymeren auf Basis von Monomeren des Typs ($\alpha$), bei denen $R^3$, $R^4$ und $R^5$ Methylgruppen sind, X eine NH-Gruppe und $A^{(-)}$ ein Halogenid-, Methoxysulfat- oder Ethoxysulfat-Ion ist; Acrylamidopropyltrimethylammoniumchlorid ist ein besonders bevorzugtes Monomeres ($\alpha$). Als Monomeres ($\beta$) für die genannten Polymeren wird bevorzugt Acrylsäure oder ein Alkalisalz der Acrylsäure, insbesondere das Natriumsalz, verwendet.

Weiterhin sind solche zwitterionischen Polymere bevorzugt, bei denen die Zahl der Monomeren vom Typ ($\alpha$) größer als die Zahl der Monomeren vom Typ ($\beta$) ist. Momomerenverhältnisse ($\alpha$):($\beta$) größer als 1,5 sind besonders bevorzugt.

Sowohl kationische als auch zwitterionische Polymere sind in den erfindungsgemäßen Zubereitungen bevorzugt in Mengen von 0,01 - 10 Gew.-%, bezogen auf die gesamte Zubereitung, enthalten. Mengen von 0,01 bis 5 Gew.-% sind besonders bevorzugt.

Zubereitungen, die kationische Polymere und zwitterionische Polymere in Mengenverhältnissen zwischen 5:1 und 1:1 enthalten, haben sich als besonders wirksam erwiesen.

Es wurde ferner gefunden, daß besonders gute Ergebnisse im Sinne der erfindungsgemäßen Lehre erzielt werden, wenn die erfindungsgemäßen Zubereitungen zusätzlich ein Tensid enthalten. Es kann sich dabei um ein anionisches, kationisches, ampholytisches, zwitterionisches oder nichtionisches Tensid handeln.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil$^R$-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid$^R$S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex$^R$ vertriebenen Dialkylammoniummethosulfate und Methylhydroxyalkyldialkoyloxyalkyl-ammoniummethosulfate.

Ein Beispiel für ein als kationisches Tensid einsetzbares quaternäres Zuckerderivat stellt das Handelsprodukt Glucquat$^R$100 dar, gemäß CTFA-Nomenklatur ein "Lauryl Methyl Gluceth-10 Hydroxypropyl Dimonium Chloride".

Besonders bevorzugte kationische Tenside sind Alkylamidoamine, quaternäre Esterverbindungen und quaternäre Zuckerderivate.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,

- lineare Fettsäuren mit 10 bis 22 C-Atomen (Seifen),
- Ethercarbonsäuren der Formel $R-O-(CH_2-CH_2O)_x-CH_2-COOH$, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel $RK-O(CH_2-CH_2O)_x-OSO_3H$, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

EP 0 648 103 B1

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, sowie Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Als zwitterionische Tenside werden solche oberflächenaktiven Verbindungen bezeichnet, die im Molekül mindestens eine quartäre Ammoniumgruppe und mindestens eine -COO$^{(-)}$- oder -SO$_3^{(-)}$-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer $C_8$-$C_{18}$-Alkyl- oder -Acylgruppe im Molekül mindestens eine freie Aminogruppe und mindestens eine -COOH- oder -SO$_3$H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das $C_{12-18}$-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- $C_{12}$-$C_{22}$-Fettsäuremono- und -diester von Anlagerungsprodukten von 1 bis 30 Mol Ethylenoxid an Glycerin,
- $C_8$-$C_{22}$-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Die Tensid-Menge, die in den erfindungsgemäßen Zubereitungen enthalten sein kann, hängt von der Art des Tensids und dem Verwendungszweck der Zubereitung ab.

Haarnachbehandlungsmittel enthalten bevorzugt kationische und/oder nichtionische Tenside, insbesondere in Mengen von 0,1 bis 10 Gew.-%, bezogen auf die gesamte Zubereitung. Mengen zwischen 0,1 und 3 Gew.-% sind besonders bevorzugt.

Haarkuren enthalten ebenfalls bevorzugt kationische und/oder nichtionische Tenside, insbesondere in Mengen von 0,1 bis 10 Gew.-%, bezogen auf die gesamte Zubereitung. Mengen zwischen 0,1 und 5 Gew.-% sind besonders bevorzugt.

Shampoos enthalten bevorzugt anionische Tenside, gewünschtenfalls in Kombination mit ampholytischen und/oder nichtionischen Tensiden, insbesondere in Mengen von 1 bis 50 Gew.-%, bezogen auf die gesamte Zubereitung. Mengen zwischen 3 und 20 Gew.-% sind besonders bevorzugt.

Die erfindungsgemäßen Zubereitungen weisen bevorzugt pH-Werte von 2,5 bis 7, insbesondere von 3 bis 6, auf.

Shampoos, Haarnachbehandlungsmittel und Haarkuren sind bevorzugte erfindungsgemäße Zubereitungen. Gleichwohl kann es sich bei den Zubereitungen auch um Dauerwellpräparate, Färbemittel, Fönwellen und andere übliche Haarbehandlungsmittel handeln.

Die Zubereitungen können als wäßrige oder wäßrig-alkoholische Lösungen, Cremes, Lotionen, Gele, Emulsionen sowie anderen, in der Kosmetik üblichen Konfektionierungsformen formuliert sein.

5

EP 0 648 103 B1

Entsprechend der Art des Haarbehandlungsmittels und der Konfektionierungsform können die Zubereitungen alle bekannten weiteren Bestandteile enthalten.

Solche üblichen Bestandteile sind:

- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butylacrylamid-Terpolymere`
- Verdickungsmittel wie Agar-Agar, Guar-Gum, Alginate, Xanthan-Gum und Cellulose-Derivate,
- Strukturanten wie Glucose und Maleinsäure,
- haarkonditionierende Verbindungen wie Phospholipide, beispielsweise Sojalecithin, Ei-Lecithin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler, wie Ethanol, Isopropanol, Ethylenglykol, Propylenglykol, Glycerin und Diethylenglykol,
- Farbstoffe,
- Antischuppenwirkstoffe wie Piroctone Olamine und Zink Omadine,
- weitere Substanzen zur Einstellung des pH-Wertes,
- Wirkstoffe wie Panthenol, Allantoin, Pflanzenextrakte und Vitamine,
- Lichtschutzmittel,
- Konsistenzgeber wie Zuckerester, Polyolester oder Polyolalkylether,
- Fette und Wachse, wie Walrat, Bienenwachs, Montanwachs, Paraffine und Fettalkohole,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, $N_2O$, Dimethylether, $CO_2$ und Luft sowie
- Antioxidantien.
- direktziehende Farbstoffe
- sogenannte Kuppler- und Entwicklerkomponenten als Oxidationsfarbstoffvorprodukte,
- Reduktionsmittel wie z.B. Thioglykolsäure und deren Derivate, Thiomilchsäure, Cysteamin, Thioäpfelsäure und $\alpha$-Mercaptoethansulfonsäure,
- Oxidationsmittel wie Wasserstoffperoxid, Kaliumbromat und Natriumbromat.

Ebenfalls Gegenstand der Erfindung ist ein Verfahren zur Behandlung von Haaren mit einer erfindungsgemäßen Zubereitung.

Bevorzugt ist ein solches Verfahren, bei dem Zubereitungen mit der erfindungsgemäßen Wirkstoffkombination auf das Haar aufgebracht werden und nach einer gewissen Einwirkzeit, die in der Regel zwischen einigen Sekunden und ca. 20 Minuten liegt, mit Wasser oder einem im wesentlichen Wasser enthaltenden Mittel wieder vom Haar abgespült werden.

Die folgenden Beispiele sollen die Erfindung näher erläutern.

**Beispiele**

**I. Bestimmung der Naßkämmbarkeit**

Untersuchungsmethoden

Die Naßkämmbarkeit wurde mit zwei verschiedenen Methoden untersucht. Methode 1 beruht auf der objektiven Bestimmung der Naßkämmarbeit, Methode 2 auf einer subjektiven Beurteilung durch Testpersonen.

Methode 1:

Den Untersuchungen zur Kämmbarkeit wurde die Methode gemäß J. Soc. Cosm. Chem. 1973 [24] 782 zugrundegelegt.

Es wurde jeweils die Kämmarbeit an braunem Haar (Alkinco #6634, Strähnenlänge 12 cm, Strähnenmasse 1 g) untersucht. Es handelte sich um leicht vorgeschädigte (kaltgewellte bzw. blondierte) Haare, wie sie etwa beim durchschnittlichen Anwender zu erwarten sind. Nach der Nullmessung wurden die Strähnen mit 100 ml der zu prüfenden Rezeptur getränkt. Nach 5 Minuten Einwirkzeit wurden die Strähnen 1 Minute unter fließendem Wasser (1 l/min, 38 °C) ausgespült. Zur Bestimmung der Naßkämmarbeit wurden die Strähnen dann erneut vermessen.

Methode 2:

Ca. 2 g schwere Haarsträhnen der Firma Kerling wurden durch jeweils eine Dauerwellbehandlung und eine "Mediumblondierung" (30 Minuten lange Behandlung der Haare mit einer 6%-igen $H_2O_2$-Lösung, die mit Ammoniak auf einen pH-Wert von 9,4 eingestellt wurde) vorgeschädigt. Anschließend wurden die Strähnen mit einer wäßrigen Lösung von Texapon N 25 (Natriumlaurylethersulfat), die auf 12 % Aktivsubstanz eingestellt war, zweimal gewaschen. Anschließend wurde die Strähne zwei Minuten lang mit 0,5 g der zu prüfenden Lösung behandelt. Zuletzt wurde mit 30 °C warmem Wasser gespült.

Ergebnisse

Die Zusammensetzung der untersuchten Mischungen sowie die Ergebnisse der Kämmbarkeitsuntersuchungen sind in Tabelle 1 zusammengestellt. Die Werte für die Kämmarbeiten nach Methode 1 sind jeweils auf den Wert der Nullmessung bezogen. Der Notenschlüssel für die subjektive Beurteilung nach Methode 2 reichte von 1 = sehr gut über 2 = gut, 3 = zufriedenstellend und 4 = ausreichend bis zu 5 = mangelhaft.

Tabelle 1

| [Mengenangaben in Gewichtsteilen] | | | | | |
|---|---|---|---|---|---|
| Komponente / Mischung | V1 | V2 | E1 | V3 | V4 |
| Celquat[R]L 200[1] | 0,1 | - | 0,05 | - | 0,05 |
| Acrylamidopropylmethylammoniumchlorid/Acrylsäure-Copolymeres, mit Natronlauge neutralisiert (Polymer P1, gemäß DE 39 29 973) | - | 0,1 | 0,05 | - | - |
| Amphomer[R] [1a] | - | - | - | 0,1 | 0,05 |
| Wasser | <----------ad 100 ----------> | | | | |
| Naßkämmarbeit | | | | | |
| - nach Methode 1 [%] | 64 | 46 | 43 | 85 | 72 |
| - nach Methode 2 | 2-3 | 2-3 | 1-2 | 3-4 | 3 |

[1] Hydroxyethylcellulose/Diallyldimethylammoniumchlorid (95 % Aktivsubstanz; CTFA-Bezeichnung: Polyquaternium-4) (Delft National)

[1a] Methylmethacrylat-tert.Butylaminoethylmethacrylat-2-Hydroxypropylmethacrylat-N(1,1,3,3-Tetramethylbutyl)acrylamid-Copolymer

**II. Bestimmung des Lockenrückhaltevermögens (Curl-Retention)**

Untersuchungsmethode

Eine 15 cm lange Haarsträhne (Fa. Kerling; Strähnengewicht: 2 g) wurde mit 0,5 g der zu untersuchenden Mischung 2 Minuten lang behandelt. Danach wurde die Strähne gespült und auf ein Glasrohr mit einem Außendurchmesser von 2,0 cm gewickelt, fixiert und getrocknet. Ein Maß für die Stabilität der nach dem Herausziehen des Glasstabes erhaltenen Locke ist der sogenannte "Curl-Retention-Wert" (CR-Wert), der definiert ist als $[(l-l_x)/(l-l_o)] * 100 \%$, wobei l die Länge der Haarsträhne (15 cm), $l_o$ die Länge der Haarlocke unmittelbar nach dem Trocknen und $l_x$ die Länge der Haarlocke nach x-stündiger hängender Lagerung in einem Trockenschrank bei konstanten Bedingungen (25 °C, 60 % relative Luftfeuchtigkeit) ist.

Ergebnisse

Die Ergebnisse der Versuche zum Lockenrückhaltevermögen sind in Tabelle 2 zusammengestellt.

Tabelle 2

| Meßwert / Mischung | V1 | V2 | E1 | V3 | V4 |
|---|---|---|---|---|---|
| CR-Werte nach | | | | | |
| - nach 6 Stunden [%] | 81,4 | 82,6 | 86,5 | 88,4 | 82,9 |
| - nach 8 Stunden [%] | 79,8 | 80,7 | 83,7 | 86,2 | 81,3 |

Die Werte für die Behandlung mit reinem Wasser betrugen 83,2 (nach 6 Stunden) bzw. 79,9 (nach 8 Stunden).

## III. Anwendungsbeispiele

Die Mengenangaben in den folgenden Beispielen sind Gew.-%.

| 1) Haarspülung | |
|---|---|
| Stenol$^R$1618[2] | 3,0 |
| Cutina$^R$GMS[3] | 0,5 |
| Eumulgin$^R$B 1[4] | 0,4 |
| Eumulgin$^R$B 2[5] | 0,8 |
| Eutanol$^R$G[6] | 1,0 |
| Acrylamidopropyltrimethylammoniumchlorid/Acrylsäure-Copolymeres, mit Natronlauge neutralisiert (Polymer P1, gemäß DE 39 29 973) | 0,2 |
| Celquat$^R$L200 | 1,0 |
| Gafquat$^R$755-N[7] | 2,5 |
| Wasser | ad 100 |

[2] C16/C18-Fettalkohol (HENKEL)
[3] Glycerinmonostearat (CTFA-Bezeichnung: Glyceryl Stearate) (HENKEL)
[4] Cetylstearylalkohol mit ca. 12 Mol EO (CTFA-Bezeichnung: Ceteareth-12) (HENKEL)
[5] Cetylstearylalkohol mit ca. 20 Mol EO (CTFA-Bezeichnung: Ceteareth-20) (HENKEL)
[6] Kondensationsprodukt aus gesättigten flüssigen Fettalkoholen, vorwiegend Decylalkohol, hergestellt nach der Guerbet-Reaktion (CTFA-Bezeichnung: Octyldodecanol) (HENKEL)
[7] Vinylpyrrolidon/Dimethylaminoethylmethacrylat-Copolymer, mit Diethylsulfat quaterniert (19 % Aktivsubstanz in Wasser) (GAF)

| 2) Haarspülung | |
|---|---|
| Stenol[R]1618 | 1,8 |
| Tegoamid[R]S 18[8] | 1,6 |
| 1,2-Propylenglykol | 1,0 |
| Zitronensäure | 0,6 |
| Celquat[R]L200 | 1,2 |
| Polymer P1, gemäß DE 39 29 973 | 0,2 |
| Parfümöl, Wasser | ad 100 |

[8] N,N-Dimethyl-N'-stearoyl-1,3-diamino-propan (CTFA-Bezeichnung: Stearamidopropyl Dimethylamin (GOLDSCHMIDT)

| 3) Haarspülung | |
|---|---|
| Stenol[R]1618 | 1,8 |
| Stepantex[R] VS 90[9] | 1,8 |
| 1,2-Propylenglykol | 0,7 |
| Zitronensäure | 0,2 |
| Celquat[R]L200 | 1,2 |
| Polymer P1, gemäß DE 39 29 973 | 0,3 |
| Parfümöl, Farbstoff, Wasser | ad 100 |

[9] N-Methyl-N(2-hydroxyethyl)-N,N-di(talgacyloxyethyl)ammoniummethosulfat (90% Aktivsubstanz in Isopropanol) (STEPAN)

| 4) Haarspülung | |
|---|---|
| Stenol[R]1618 | 2,5 |
| Eumulgin[R]B 1 | 1,0 |
| Eumulgin[R]B 2 | 1,0 |
| Cutina[R]CP[10] | 1,0 |
| Eutanol[R]G | 0,5 |
| Polawax[R]GP 200[11] | 0,75 |
| Abil[R]Quat 3270[12] | 0,5 |
| Dow Corning[R]929-Emulsion[13] | 2,6 |
| Polymer JR[R]400[14] | 0,6 |
| Polymer P1, gemäß DE 39 29 973 | 0,4 |
| Zitronensäure | 0,02 |
| Wasser | ad 100 |

[10] Ester aus gesättigten, langkettigen Fettalkoholen und Fettsäuren, vornehmlich Palmitinsäurecetylester (CTFA-Bezeichnung: Cetyl Palmitate) (HENKEL)
[11] Stearylalkohol-Polyethylenglykolstearat-Gemisch (CTFA-Bezeichnung: Stearyl Alkohol (and) PEG-Stearate) (CRODA)
[12] Diquaternäres Polymethylsiloxan (CTFA-Bezeichnung: Ouaternium-80) (GOLDSCHMIDT)
[13] amino-funktionelles Polydimethylsiloxan (35 % Aktivsubstanz) (DOW CORNING)
[14] quaternierte Hydroxyethylcellulose (CTFA-Bezeichnung: Polyquaternium-10) (Union Carbide)

| 5) Haarspülung | |
|---|---|
| Rewoquat[R]W 7500[15] | 1,1 |
| Lanette[R]O[16] | 3,0 |
| Eumulgin[R]B 1 | 0,8 |
| Eumulgin[R]B 2 | 1,6 |
| Cutina[R]GMS | 0,5 |
| Eutanol[R]G | 1,0 |
| Polymer JR[R]-125[17] | 1,0 |
| Polymer P1, gemäß DE 39 29 973 | 0,4 |
| Wasser | ad 100 |

[15] 1-Methyl-2-nortalgalkyl-3-talgfettsäure-amidoethyl-imidazoliniummethosulfat (ca. 75 % Aktivsubstanz) (REWO)
[16] Gemisch höherer, gesättigter Fettalkohole, vorwiegend Cetyl- und Stearylalkohol (CTFA-Bezeichnung: Cetearyl Alcohol) (HENKEL)
[17] quaternierte Hydroxyethylcellulose (CTFA-Bezeichung: Polyquaternium-10) (Union Carbide)

| 6) Shampoo | |
|---|---|
| Texapon[R]N 25[18] | 43,0 |
| Dehyton[R]K[19] | 10,0 |
| Plantaren[R]-1200[20] | 4,0 |
| Euperlan[R]PK 3000[21] | 1,6 |
| Arquad[R]316[22] | 0,5 |
| Celquat[R]L200 | 1,2 |
| Polymer P1, gemäß DE 39 29 973 | 0,2 |
| Glucamate[R]DOE 120[23] | 0,5 |
| Natriumchlorid | 0,2 |
| Wasser | ad 100 |

[18] Natriumlaurylethersulfat (ca. 28 % Aktivsubstanz; CTFA-Bezeichnung: Sodium Laureth Sulfate) (HENKEL)

[19] Fettsäureamid-Derivat mit Betainstruktur der Formel $R\text{-}CONH(CH_2)_3N^+(CH_3)_2CH_2COO^-$ (ca. 30 % Aktivsubstanz; CTFA-Bezeichnung Cocoamidopropyl Betaine) (HENKEL)

[20] C12-C16-Alkylglucosid mit Oligomerisationsgrad 1,4 (ca. 50 % Aktivsubstanz; CTFA-Bezeichnung: Lauryl Polyglycosid) (HENKEL)

[21] Flüssige Dispersion von perlglanzgebenden Substanzen und Amphotensid (ca. 62 % Aktivsubstanz; CTFA-Bezeichnung: Glycol Distearate (and) Glycerin (and) Laureth-4 (and) Cocoamidopropyl Betaine) (HENKEL)

[22] Tri-C16-alkylmethylammoniumchlorid (AKZO)

[23] ethoxyliertes Methylglucosid-dioleat (CTFA-Bezeichnung: PEG-120 Methyl Glucose Dioleate) (AMERCHOL)

| 7) Shampoo | |
| --- | --- |
| Texapon[R]N 70[24] | 21,0 |
| Plantaren[R]-1200 | 8,0 |
| Genamin[R]DSAC[25] | 1,2 |
| Cutina[R]EGMS[26] | 0,6 |
| Polymer JR[R]-400 | 0,8 |
| Polymer P1, gemäß DE 39 29 973 | 0,3 |
| Antil[R]141 liquid[27] | 1,3 |
| Natriumchlorid | 0,2 |
| Wasser | ad 100 |

[24] Natriumlaurylethersulfat (ca. 72 % Aktivsubstanz) (HENKEL)
[25] Dimethyldistearylammoniumchlorid (HOECHST)
[26] Ethylenglykolmonostearat (ca. 25-35% Monoester, 60-70% Diester; CTFA-Bezeichnung: Glycol Stearate) (HENKEL)
[27] Polyoxyethylen-propylenglykoldioleat (40 % Aktivsubstanz; CTFA-Bezeichnung: Propylene Glycol (and) PEG-55 Propylene Glycol Oleate) (GOLDSCHMIDT)

| 8) Shampoo | |
| --- | --- |
| Texapon[R]K 14 S[28] | 50,0 |
| Dehyton[R]K | 10,0 |
| Akypo[R]RLM 100 NV[29] | 4,5 |
| Cutina[R]AGS[30] | 2,0 |
| D-Panthenol | 0,5 |
| Glucose | 1,0 |
| Salicylsäure | 0,4 |
| Natriumchlorid | 0,5 |
| Polymer P1, gemäß DE 39 29 973 | 0,6 |
| Celquat[R]L200 | 0,6 |
| Wasser | ad 100 |

[28] Natriumlaurylmyristylethersulfat (ca. 28 % Aktivsubstanz; CTFA-Bezeichnung: Sodium Myreth Sulfate) (HENKEL)
[29] C12-14-Fettalkohol+10 Ethylenoxid-essigsäure-Natriumsalz (22 % Aktivsubstanz; CTFA-Bezeichnung: Sodium Laureth-11 Carboxylate) (CHEM-Y)
[30] Ethylenglykolstearat (ca. 5-15% Monoester, 85-95% Diester; CTFA-Bezeichnung: Glycol Distearate) (HENKEL)

| 9) Shampoo | |
|---|---|
| Texapon[R]K 14 S | 25,0 |
| Texapon[R]SB 3[31] | 7,5 |
| Eucarol[R]TA[32] | 12,0 |
| Akypo[R]RLM 100 NV | 9,0 |
| Dehyton[R]AB 30[33] | 8,3 |
| Elfacos[R]GT 282S[34] | 0,5 |
| Natriumchlorid | 0,5 |
| Polymer P1, gemäß DE 39 29 973 | 0,6 |
| Polymer JR[R]-400 | 2,0 |
| Wasser | ad 100 |

[31] Sulfobernsteinsäurehalbester auf Basis eines Alkylpolyglycolethers, Di-Na-Salz (ca. 40 % Aktivsubstanz; CTFA-Bezeichnung: Disodium Laurethsulfosuccinat) (HENKEL)
[32] Laurylalkohol+7 Ethylenoxid-tartrat-Natriumsalz (ca. 25 % Aktivsubstanz; CTFA-Bezeichnung: Sodium Laureth-7 Tartrate) (AUSICHEM)
[33] Fettamin-Derivat mit Betainstruktur (ca. 30 % Aktivsubstanz; CTFA-Bezeichnung: Coco-Betaine) (HENKEL)
[34] Talgalkohol+60 Ethylenoxid-myristylether (CTFA-Bezeichnung: Talloweth-60 Myristyl Glycol) (AKZO)

| 10) Kurpackung (abspülbar) | |
|---|---|
| Stenol[R]1618 | 3,0 |
| Eumulgin[R]B 1 | 0,5 |
| Eumulgin[R]B 2 | 0,5 |
| Cutina[R]CP | 1,0 |
| Eutanol[R]G | 1,5 |
| Carbopol 980[35] | 0,004 |
| Dow Corning[R]929-Emulsion | 2,9 |
| Polymer P1, gemäß DE 39 29 973 | 0,3 |
| Celquat[R]L200 | 2,0 |
| Triethanloamin | 0,09 |
| Wasser | ad 100 |

[35] Polyacrylsäure (GOODRICH)

| 11) Kurpackung (auf dem Haar verbleibend) | |
|---|---|
| Celquat[R]L 200 | 0,6 |
| Luviskol[R]K30[36] | 0,2 |
| D-Panthenol | 0,5 |
| Dehyquart[R]SP[37] | 1,0 |
| Nutrilan[R]I[38] | 1,0 |
| Natrosol[R]250 HR[39] | 1,1 |
| Polymer P1, gemäß DE 39 29 973 | 0,5 |
| Wasser | ad 100 |

[36] Polyvinylpyrrolidon (95 % Aktivsubstanz; CTFA-Bezeichnung: PVP) (BASF)
[37] Wäßrige Lösung von Oxyethylalkylammonium-phosphat (ca. 50 % Aktivsubstanz; CTFA-Bezeichnung: Ouaternium-52) (HENKEL)
[38] Kollagenhydrolysat (ca. 39 % Aktivsubstanz; CTFA-Bezeichnung: Hydrolyzed Collagen) (HENKEL)
[39] Hydroxyethylcellulose (AOUALON)

| 12) Färbecreme | |
|---|---|
| C12-18-Fettalkohol | 1,2 |
| Lanette[R]O | 4,0 |
| Eumulgin[R]B 2 | 0,8 |
| Cutina[R]KD 16[40] | 2,0 |
| Natriumsulfit | 0,5 |
| L(+)-Ascorbinsäure | 0,5 |
| Ammoniumsulfat | 0,5 |
| 1,2-Propylenglykol | 1,2 |
| p-Aminophenol | 0,35 |
| p-Toluylendiamin | 0,85 |
| 2-Methylresorcin | 0,14 |
| 6-Methyl-m-aminophenol | 0,42 |
| Polymer P1, gemäß DE 39 29 973 | 0,5 |
| Celquat[R]L200 | 1,5 |
| Ammoniak | 1,5 |
| Wasser | ad 100 |

[40] Fettsäuremono/diglycerid-Emulgator-Gemisch (CTFA-Bezeichnung: Tallow Glycerides (and) Glyceryl Stearate (and) Potassium Stearate) (HENKEL)

| 13) Entwickleremulsion für Färbecreme 12) | |
|---|---|
| Texapon$^R$N 25 | 2,1 |
| Wasserstoffperoxid (50%ig) | 12,0 |
| Turpinal$^R$SL[41] | 1,7 |
| Latekoll$^R$D[42] | 12,0 |
| Polymer P1, gemäß DE 39 29 973 | 0,1 |
| Polymer JR$^R$-400 | 0,1 |
| Wasser | ad 100 |

[41] 1-Hydroxyethan-1,1-diphosphonsäure (60 % Aktivsubstanz; CTFA-Bezeichnung: Etidronic Acid) (HENKEL)
[42] Acrylester-Methacrylsäure-Copolymer (25 % Aktivsubstanz) (BASF)

Die Färbecreme hatte einen pH-Wert von 10,0. Sie bewirkte eine intensive rote Tönung des Haares.

| 14) Tönungsshampoo | |
|---|---|
| Texapon$^R$N 70 | 14,0 |
| Dehyton$^R$K | 10,0 |
| Akypo$^R$RLM 45 NV[43] | 14,7 |
| Plantaren$^R$-1200 | 4,0 |
| Cremophor$^R$RH 40[44] | 0,8 |
| Farbstoff C.I. 12 719 | 0,02 |
| Farbstoff C.I. 12 251 | 0,02 |
| Farbstoff C.I. 12 250 | 0,04 |
| Farbstoff C.I. 56 059 | 0,03 |
| PHB-Ester | 0,25 |
| Celquat$^R$L200 | 0,8 |
| Polymer P1, gemäß DE 39 29 973 | 0,2 |
| Parfümöl | q.s. |
| Wasser | ad 100 |

[43] Laurylalkohol+4,5 Ethylenoxid-essigsäure-Natriumsalz (20,4 % Aktivsubstanz) (CHEM-Y)
[44] Rizinus-Öl, hydriert + 45 Ethylenoxid (CTFA-Bezeichnung: PEG-40 Hydrogenated Castor Oil) (BASF)

Beim Waschen der Haare mit diesem Tönungs-Shampoo erhalten diese einen glänzenden, hellblonden Farbton.

EP 0 648 103 B1

| 15) Cremedauerwelle | |
|---|---|
| Wellcreme: | |
| Plantaren$^R$-800$^{45}$ | 5,0 |
| Thioglykolsäure | 8,0 |
| Turpinal$^R$SL | 0,5 |
| Ammoniak (25%ig) | 7,3 |
| Ammoniumcarbonat | 3,0 |
| Cetyl/Stearyl-Alkohol | 5,0 |
| Guerbet-Alkohol | 4,0 |
| Celquat$^R$L200 | 1,2 |
| Polymer P1, gemäß DE 39 29 973 | 1,0 |
| Parfümöl | q.s. |
| Wasser | ad 100 |
| Fixierlösung: | |
| Plantaren$^R$-800 | 5,0 |
| gehärtetes Rizinusöl | 2,0 |
| Kaliumbromat | 3,5 |
| Nitrilotriessigsäure | 0,3 |
| Zitronensäure | 0,2 |
| Polymer P1, gemäß DE 39 29 973 | 0,4 |
| Polymer JR$^R$-400 | 1,0 |
| Parfümöl | q.s. |
| Wasser | ad 100 |

$^{45}$ C8-C10-Alkylglucosid mit Oligomerisationsgrad 1,6 (ca. 60% Aktivsubstanz) (HENKEL)

**Patentansprüche**

1. Wäßrige Zubereitung zur Behandlung von Haaren, enthaltend neben üblichen kosmetischen Bestandteilen eine Kombination von kationischen und zwitterionischen Polymeren, dadurch gekennzeichnet, daß die kationischen Polymere Cellulose-Derivate sind und keine Imidazoliniumgruppen enthalten und sich die zwitterionischen Polymeren im wesentlichen zusammensetzen aus

α) Monomeren mit quartären Ammoniumgruppen der allgemeinen Formel (I),

$$R^1\text{-CH=CR}^2\text{-CO-Z-}(C_nH_{2n})\text{-N}^{(+)}R^3R^4R^5 \; A^{(-)} \qquad \text{(I)}$$

in der $R^1$ und $R^2$ unabhängig voneinander stehen für Wasserstoff oder eine Methylgruppe und $R^3$, $R^4$ und $R^5$ unabhängig voneinander für Alkylgruppen mit 1 bis 4 Kohlenstoff-Atomen, Z eine NH-Gruppe oder ein Sauerstoffatom, n eine ganze Zahl von 2 bis 5 und $A^{(-)}$ das Anion einer organischen oder anorganischen Säure ist. und

β) monomeren Carbonsäuren der allgemeinen Formel (II),

$$R^6\text{-CH=CR}^7\text{-COOH} \qquad \text{(II)}$$

17

EP 0 648 103 B1

in denen R$^6$ und R$^7$ unabhängi voneinander Wasserstoff oder Methylgruppen sind, oder Alkali-, Erdalkali-, Aluminium- oder Ammoniumsalzen dieser Säuren.

2. Zubereitung nach Anspruch 1, dadurch gekennzeichnet, daß das kationische Polymere ein Copolymeres aus mindestens einer kationischen Einheit und mindestens einer nichtionischen Einheit ist.

3. Zubereitung nach Anspruch 2, dadurch gekennzeichnet, daß die nichtionische Einheit Hydroxyethylcellulose ist.

4. Zubereitung nach einem der Ansprüche 1 bis 3, dadurch gekennzeichnet, daß das Monomere des Typs (α) Acrylamidopropyl-trimethyl-ammoniumchlorid und das Monomere des Typs (β) Acrylsäure oder ein Alkalisalz, insbesondere das Natriumsalz, der Acrylsäure ist.

5. Zubereitung nach einem der Ansprüche 1 bis 4, dadurch gekennzeichnet, daß im zwitterionischen Polymer die Zahl der Monomeren vom Typ (α) größer als die Zahl der Monomeren vom Typ (β) ist.

6. Zubereitung nach einem der Ansprüche 1 bis 5, dadurch gekennzeichnet, daß sie

   - 0,01 — 10, insbesondere 0,01 bis 5 Gew.-%, an zwitterionischen Polymeren und
   - 0,01 — 10, insbesondere 0,01 bis 5 Gew.-%, an kationischen Polymeren,

   jeweils bezogen auf die gesamte Zubereitung, enthält.

7. Zubereitung nach einem der Ansprüche 1 bis 6, dadurch gekennzeichnet, daß sie zusätzlich ein Tensid enhält.

8. Zubereitung nach Anspruch 7, dadurch gekennzeichnet, daß das Tensid ein kationisches und/oder nichtionisches Tensid ist.

9. Zubereitung nach einem der Ansprüche 1 bis 8, dadurch gekennzeichnet, daß die Zubereitung einen pH-Wert zwischen 2,5 und 7, insbesondere zwischen 3,0 und 6,0 aufweist.

10. Verfahren zur Behandlung von Haaren, dadurch gekennzeichnet, daß eine Zubereitung nach einem der Ansprüche 1 bis 9 auf das Haar aufgebracht wird.

11. Verfahren nach Anspruch 10, dadurch gekennzeichnet, daß das Haar anschließend mit Wasser oder einem im wesentlichen Wasser enthaltenden Mittel gespült wird.

**Claims**

1. A water-based hair treatment preparation containing a combination of cationic and zwitterionic polymers in addition to typical cosmetic constituents, characterized in that the cationic polymers are cellulose derivatives and do not contain any imidazolinium groups and in that the zwitterionic polymers consist essentially of

   (α) monomers containing quaternary ammonium groups corresponding to general formula (I):

   $$R^1\text{-CH=CR}^2\text{-CO-Z-}(C_nH_{2n})\text{-N}^{(+)}R^3R^4R^5 \; A^{(-)} \tag{I}$$

   in which R$^1$ and R$^2$ independently of one another represent hydrogen or a methyl group and R$^3$, R$^4$ and R$^5$ independently of one another represent C$_{1-4}$ alkyl groups, Z is an NH group or an oxygen atom, n is an integer of 2 to 5 and A$^{(-)}$ is the anion of an organic or inorganic acid
   and
   (β) monomeric carboxylic acids corresponding to general formula (II):

   $$R^6\text{-CH=CR}^7\text{-COOH} \tag{II}$$

   in which R$^6$ and R$^7$ independently of one another are hydrogen or methyl groups,
   or alkali metal, alkaline earth metal, aluminium or ammonium salts of these acids.

2. A preparation as claimed in claim 1, characterized in that the cationic polymer is a copolymer of at least one cati-

18

onic unit and at least one nonionic unit.

3. A preparation as claimed in claim 2, characterized in that the nonionic unit is hydroxyethyl cellulose.

4. A preparation as claimed in any of claims 1 to 3, characterized in that the monomer ($\alpha$) is acrylamidopropyl trimethyl ammonium chloride while the monomer ($\beta$) is acrylic acid or an alkali metal salt, particularly the sodium salt, of acrylic acid.

5. A preparation as claimed in any of claims 1 to 4, characterized in that the number of monomers ($\alpha$) in the zwitterionic polymer is greater than the number of monomers ($\beta$).

6. A preparation as claimed in any of claims 1 to 5, characterized in that it contains

   - 0.01 to 10% by weight and more particularly 0.01 to 5% by weight of zwitterionic polymers and
   - 0.01 to 10% by weight and more particularly 0.01 to 5% by weight of cationic polymers,

   based on the preparation as a whole.

7. A preparation as claimed in any of claims 1 to 6, characterized in that it also contains a surfactant.

8. A preparation as claimed in claim 7, characterized in that the surfactant is a cationic and/or nonionic surfactant.

9. A preparation as claimed in any of claims 1 to 8, characterized in that the preparation has a pH value of 2.5 to 7 and, more particularly, in the range from 3.0 to 6.0.

10. A process for treating hair, characterized in that a preparation as claimed in any of claims 1 to 9 is applied to the hair.

11. A process as claimed in claim 10, characterized in that the hair is subsequently rinsed with water or with a preparation essentially containing water.

## Revendications

1. Préparation aqueuse pour le traitement des cheveux contenant à côté des constituants cosmétiques usuels, une combinaison de polymères cationiques et zwitterioniques,
   caractérisée en ce que
   les polymères cationiques sont des dérivés de la cellulose et ne contiennent aucun groupe imidazolinium et en ce que les polymères zwitterioniques se composent pour l'essentiel de :

   $\alpha$) monomères avec des groupes ammonium quaternaires de formule générale (I),

   $$R^1\text{-}CH{=}CR^2\text{-}CO\text{-}Z\text{-}(C_nH_{2n})\text{-}N^{(+)}R^3R^4R^5\ A^{(-)} \tag{I}$$

   dans laquelle $R^1$ et $R^2$ indépendamment l'un de l'autre représentent de l'hydrogène ou un radical méthyle et $R^3$, $R^4$ et $R^5$ indépendamment l'un de l'autre représentent des radicaux alkyle ayant de 1 à 4 atomes de carbone, Z un groupe -NH- ou un atome d'oxygène, n est un nombre entier allant de 2 à 5, et $A^{(-)}$ est l'anion d'un acide organique ou minéral,
   et
   $\beta$) des acides carboxyliques monomères de formule générale (II),

   $$R^6\text{-}CH{=}CR^7\text{-}COOH \tag{II}$$

   dans laquelle $R^6$ et $R^7$ indépendamment l'un de l'autre sont de l'hydrogène ou un radical méthyle ou bien les sels de métal alcalin, de métal alcalino-terreux, d'aluminium ou d'ammonium de ces acides.

2. Préparation selon la revendication 1,
   caractérisée en ce que
   le polymère cationique est un copolymère à base d'au moins un élément cationique et d'au moins un élément non ionique.

3. Préparation selon la revendication 2,
caractérisée en ce que
l'élément non ionique est l'hydroxyéthylcellulose.

4. Préparation selon l'une des revendications 1 à 3,
caractérisée en ce que
le monomère du type ($\alpha$) est le chlorure d'acrylamidopropyltriméthylammonium et le monomère du type ($\beta$) est l'acide acrylique ou un sel de métal alcalin, en particulier le sel de sodium.

5. Préparation selon l'une des revendications 1 à 4,
caractérisée en ce que
dans le polymère zwitterionique le nombre des monomères du type ($\alpha$) est plus grand que le nombre des monomères du type ($\beta$).

6. Préparation selon l'une des revendications 1 à 5,
caractérisée en ce qu'
elle renferme de 0,01 à 10, en particulier de 0,01 à 5 % en poids de polymères zwitterioniques et de 0,01 à 10, en particulier de 0,01 à 5 % en poids de polymères cationiques, à chaque fois rapporté à la préparation totale.

7. Préparation selon l'une des revendications 1 à 6,
caractérisée en ce qu'
elle renferme en supplément un agent tensioactif.

8. Préparation selon la revendication 7 ,
caractérisée en ce que
l'agent tensioactif est un agent tensioactif cationique et/ou non ionique.

9. Préparation selon l'une des revendications 1 à 8,
caractérisée en ce que
la préparation possède une valeur de pH comprise entre 2,5 et 7, en particulier entre 3,0 et 6,0.

10. Procédé de traitement des cheveux,
caractérisé en ce qu'
une préparation selon l'une des revendications 1 à 9 est appliquée sur les cheveux.

11. Procédé selon la revendication 10,
caractérisé en ce que
les cheveux sont rincés ensuite avec de l'eau ou avec une composition renfermant essentiellement de l'eau.